(19) Europäisches Patentamt
European Patent Office
Office européen des brevets

(11) EP 2 857 067 A1

(12) EUROPEAN PATENT APPLICATION

(43) Date of publication:
**08.04.2015 Bulletin 2015/15**

(21) Application number: **14187604.5**

(22) Date of filing: **03.10.2014**

(51) Int Cl.:
***A61P 17/16** (2006.01)* ***A61K 36/28** (2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **04.10.2013 IT RM20130541**

(71) Applicant: **Aboca S.p.A. Societa' Agricola**
**52037 Sansepolcro (AR) (IT)**

(72) Inventors:
- **Mercati, Valentino**
  **52037 Sansepolcro AR (IT)**
- **Mattoli, Luisa**
  **52037 Sansepolcro AR (IT)**

(74) Representative: **Predazzi, Valentina et al**
**Società Italiana Brevetti S.p.A.**
**Piazza di Pietra, 39**
**00186 Roma (IT)**

# (54) Grindelia extracts depleted in grindelic acid and uses thereof

(57) The present invention relates to new fractions of grindelia extracts in which only traces of grindelic acid, saponins and resins are present, a process for the preparation of such fractions, compositions comprising them, and use thereof for the protection or for cooperating with the protection of skin or mucous membranes.

EP 2 857 067 A1

## Description

The present invention relates to specific fractions of grindelia (tops) extract having mucoadhesive and/or barrier effect properties, in which very low concentrations of grindelic acids, and optionally of saponins, are present, a process for the preparation of said fractions, compositions comprising it, and the use of the fractions and of the compositions for the protection or for cooperating with the protection of skin or mucous membranes.

## STATE OF THE PRIOR ART

Grindelia is the name generally given to plant Grindelia (*Grindelia robusta* and *Grindelia squarrosa*); it is a flowering plant native of South America and also grown in Europe. The part used for medical purposes is comprised of the flowering tops with leaves. It is commonly used for the treatment of airway inflammations with mucus hypersecretion.

Grindelia contains resins characterised by a high content of terpenoid substances such as grindelic acid, and markedly haemolytic triterpenic saponins as main active principles for the therapeutic applications for which it is commonly used.

Commonly, the fluid and dry extracts of grindelia obtained by hydroalcoholic extraction contain grindelic acid, and saponins, corresponding in the dry extract to about 10% by weight of grindelic acid and about 0.2% of saponins, and the various known extraction methods are focused on improving the yield in these components or on making such components more useful for therapeutic purposes. At the above-indicated concentrations of saponins, a foam index equal to 700 and a positive foam test are referable. The foam index is a standard measurement performed on extracts with a positive foam test. The Foam Test is a simple and general qualitative test used as a first indicator of saponins presence in plant extracts. Many drugs contain saponins able to form a lasting foam when their aqueous extracts are stirred. The ability of an aqueous extract of a plant substance and of its extracts to lastingly form foam is denoted as "foam index".

In the literature it is reported how mucoadhesive materials can be useful for therapeutic purposes or to carry drugs, or as therapeutic agents as such, coating and protecting damaged tissues (for instance, gastric ulcers or lesions of the mucous membranes) (J.D. Smart 2005 Advanced drug delivery reviews 57 pp 1556-1568).

Given the medical interest for mucoadhesive substances and for those of natural origin, it is useful to develop novel natural mucoadhesive compounds to be used as drug carriers or for a therapeutic/protective aim.

## SUMMARY OF THE INVENTION

The Authors of the present invention have identified mucoadhesive substances, having a barrier effect, of natural origin which are advantageously depleted in active principles which would not enable their general use as drug carriers or as agents for a prevention/therapy of damaged or irritated mucous membranes due to a mechanical protective barrier or coating effect thereof. The present invention relates to selected fractions of grindelia tops extract substantially depleted in the known pharmacologically active components represented by grindelic acid and optionally also in saponins, of which only traces remain, characterised by a barrier effect and mucoadhesive properties greater than those of a common reference hydroalcoholic grindelia extract, to compositions comprising such fractions and to processes for obtaining them. The fractions according to the invention may be used for a purpose completely different from that for which the grindelia extract is used in the state of the art; said use could be for the protection of the mucous membranes or of the skin, to cooperate with such protection and/or also to retain desired substances on the mucous membranes thanks to the mucoadhesive and/or barrier effect abilities of the selected fractions. The fractions of the invention therefore provide a new source of material of plant origin for use in the protection of the mucous membranes and of the skin and/or also as excipient capable of retaining for a longer time desired substances on the mucous membranes, wherein the pharmacological effects attributable to the components known as active principles, are substantially reduced or even eliminated.

Said condition is particularly desirable when materials of natural or plant origin are to be used for actions of a mainly mechanical type (i.e., not for pharmacological actions having, for instance, a direct effect on the activation of receptorial, immune pathways or metabolic processes). The use of substances having a mainly mechanical effect, such as, e.g., substances having a barrier or mucoadhesive effect, is desirable, for instance, to cooperate with a pharmacological therapy. By minimizing the presence of substances with pharmacological activity in the material of plant or natural origin as in present invention, it is obtained a material with a mainly mechanical effect that can be used in combination with drugs, minimizing possible undesired interactions with drugs, at the same time exploiting the typical feature of substances of plant origin, of having a vast number of components capable of exerting a protective effect besides the pharmacologically active ones commonly used.

The use of fractions of plant or natural extracts depleted in one or both of the most known pharmacologically active principles such as grindelic acid and saponins, and enriched in the remaining substances, entails the dual advantage of providing a material with a mainly mechanical protective action (mucous membrane protection or skin protection) and of enabling a concrete and useful use of materials normally discarded in extraction processes of plant or natural active principles.

The present invention therefore relates to fractions of grindelia tops extracts depleted in grindelic acid, and optionally in saponins, with a grindelic acid concentration of ≤0.5% or even of ≤0,2% and, optionally, wherein the foam index is of ≤250, characterised by a barrier effect measured by cell marker release inhibition assay in response to an inflammatory or irritating agent higher than or equal to 40%; their use as protective agents of the skin or mucous membranes and/or as excipients with a high mucoadhesive ability; compositions comprising such fractions; their use in the protection of the skin or mucous membranes and/or for the carrying of desired active principles on the mucous membranes and processes for the preparation of such fractions and of such compositions.

The invention also relates to a process for the preparation of fractions of grindelia extracts as defined above, comprising the following step:

a. preparing a hydroalcoholic grindelia tops extract and subjecting said extract to centrifugation and/or decantation, obtaining a precipitated fraction and a clarified alcoholic extract.
b. obtaining from the clarified alcoholic extract prepared in a. a concentrated aqueous extract,
c. decanting and/or centrifuging and filtering said aqueous extract, collecting a water-soluble fraction C with a grindelic acid concentration of ≤ 0.5%.

Moreover, the process as described above could further comprise the following step: d. subjecting to membrane ultrafiltration the fraction obtained in c. and collecting a fraction D, corresponding to the permeate obtained by said ultrafiltration, characterised by a foam index ≤250 and a grindelic acid concentration of ≤0.2%. The invention also relates to the preparation of mixtures of said fractions C and/or D with one or more natural extracts and/or active principles deriving from sources different from grindelia, such mixtures, as well as the preparation of pharmaceutical compositions or of medical foods containing one or more of said fractions in combination with other natural substances and/or active principles deriving from sources different from grindelia and the use of such fractions alone or mixed between them or with other components, for the protection of the mucous membranes and of the skin or as carriers capable of retaining desired substances (deriving from sources different from grindelia) on the mucous membranes by virtue of their effective mucoadhesive properties.

The invention therefore relates to fractions of grindelia extracts and their uses for the protection of the mucous membranes or of the skin or as excipients with good mucoadhesive properties, substantially free from the effects attributable to the commonly used active principles of grindelia, i.e. grindelic acid and saponins.

## DETAILED DESCRIPTION OF THE FIGURES

Figure 1 shows a block diagram of an embodiment of the process of the invention in which step d. is not present.
Figure 2 shows a block diagram of step d.
Figure 3 shows a drawing depicting grindelia flowering tops.

## SHORT GLOSSARY

NMWCO=Nominal Molecular Weight Cut-off

PERMEATE: extract that has passed through a semi-permeable filtration or ultrafiltration membrane.

RETENTATE: extract that did not pass through a semi-permeable filtration or ultrafiltration membrane or material adsorbed on resin.

ELUATE: material not adsorbed on resin following adsorption passages thereon.

ULTRAFILTRATION: filtration technique on semi-permeable membrane characterised by pores having sizes of 100000 daltons (0.1 μm) to 1000 daltons (about 0.005 μm)

Grindelia tops: by grindelia tops, or grindelia flowering tops it is meant the term as commonly used in herbal medicine and in botanical treatises, therefore the aerial ends of the plant which contain leaves, stems (meant as branches and not merely as main stalk of the plant) and grindelia flowers, or at least one of these components are meant, the plant being usually harvested when stalk size is at least of 30 cm in height.

GRINDELIC ACID % is a measurement of the percentage of grindelic acid in the fraction of interest by GC-MS analysis (by means of a Gas Chromatograph equipped with a single-quadripole as detector). In the chromatogram of the sample, the area corresponding to the grindelic acid, protected as trimethylsilyl derivative, is compared to the total area of the peaks present in the chromatogram itself. Quantitative information on grindelic acid content is obtained. This

analytic approach is known to the technician in the field as commonly used to obtain quantitative on, e.g., essential oils. Therefore, by grindelic acid % it is meant a concentration of grindelic acid calculated as percentage area of the total area of the GC-MS chromatogram of the analyzed fraction.

FOAM INDEX test, to highlight drugs and/or their extracts and drugs containing saponins capable of forming a lasting foam when their aqueous extracts are stirred.

FOAM TEST: simple and general qualitative test used as first indicator of saponins presence in plant extracts. These substances are foam-generating in an aqueous solution, i.e. have surfactant properties. When in an aqueous solution, they align perpendicularly with the surface, with the effect of reducing the surface tension of water and of forming foam during the stirring, The surfactant ability is highlighted by the formation of an emulsion when an oil, such as olive oil, is added to the aqueous saponins extract.

By "NOT GREATER THAN", with respect to the amounts of active principles in the extract, in the present description it is meant ≤.

The reference total extract or reference extract in the present invention is represented by the extract lyophilisate in 60% ethanol of grindelia tops as defined above.

## DETAILED DESCRIPTION OF THE INVENTION

As indicated above, in the present description there are provided fractions of grindelia extracts depleted in grindelic acid, and optionally also in saponins, with a grindelic acid concentration of ≤0.5%, or even of ≤0.2%, wherein the foam index may optionally be brought to ≤250, having good mucoadhesive and/or barrier effect abilities on the mucous membranes. The barrier effect of the fractions of the invention, when measured by cell marker release inhibition assay in response to an inflammatory or irritating agent will have a value higher than or equal to 40%. A foam index equal to about 250 corresponds to a saponins percentage of about 0.07% of saponins, with respect to the 0.2% normally present in hydroalcoholic grindelia extracts, (in fact, if initially to a 0.2 % concentration of saponins in accordance with the literature there corresponds a foam index of 700, to a foam index of 250 there corresponds a 0.07%).

By applying the fractioning processes described below, it was possible to obtain fractions of grindelia extracts depleted in grindelic acid, and optionally in saponins, and concomitantly enriched in the remaining substances, which exhibited an effective protective action on the skin and on the mucous membranes, as evident from the mucoadhesivity assays performed on cells and/or from the barrier assays reported in the experimental section below.

The fractions of the invention do not correspond to mere dilutions of a common hydroalcoholic grindelia extract (also indicated herein as reference extract) since, as can be seen in Table 2 below, the reduction factors between different components are not the same.

With respect to the dry or fluid grindelia extracts and grindelia commonly used, which comprise an amount of grindelic acid of about 10%, the fractions of the present invention comprise amount of grindelic acid of ≤0.5% or even of ≤ 0.2%. Such fractions may also be depleted in saponins. The total hydroalcoholic grindelia extract normally has a foam index greater than 700, the fractions of the present invention may have, in case, e.g., of fraction D, traces of grindelic acid and saponins, since the grindelic acid percent is ≤0.2% and the foam index is ≤250 (a value for which, with respect to the reference extract, the assayed extract volume has to be tripled in order to have a lasting foam after 30 minutes). The fractions according to the present description exhibit surprising mucoadhesive and/or barrier effect activities.

Evidently, such values of grindelic acid correspond in practice to traces of grindelic acid, and a foam index value as reported above corresponds it also to a very drastic reduction in saponins. Such values cannot be held accountable for the mucoadhesive and barrier properties exhibited by the fractions of the invention and cannot induce the pharmacological activities for which grindelia or grindelia extracts are commonly used.

The selected fractions according to the present description, substantially depleted in active principles like saponins, grindelic acid and total phenols, preserve however good mucoadhesive and/or barrier effect properties as can be seen in Table 1 below.

The table also shows mucoadhesive and barrier properties of a total hydroalcoholic grindelia extract used as reference.

The assessment of mucoadhesive properties was carried out herein by cell adhesion assay and by adhesion assay with mucin on an inclined plane described in detail in the experimental section below. The barrier effect was instead measured by cell marker release inhibition assay in response to an inflammatory or irritating agent, as described in detail in the experimental section below.

Table 1

| Fraction | Barrier effect assay NET BARRIER EFFECT: % of IL-6 inhibition of the B.A. -% of IL-6 inhibition of the IC | Cell adhesion assay -% | Mucoadhesion assay on inclined plane % |
|---|---|---|---|
| REFERENCE GRINDELIA EXTRACT, LYOPHILIZED | 32 | 64 | 61 |
| GRINDELIA FRACTION C, LYOPHILIZED | 73 | 69 | n.d. |
| GRINDELIA FRACTION D, GE PERMEATE <1000 DA, LYOPHILIZED | **43** | **73** | **86** |

In the present invention, the mucoadhesion percentage could be measured according to any method known to a technician in the field. In particular, the mucoadhesion percentage can be measured according to a simple mucoadhesion assay on an inclined plane that is explained in a detailed manner in the experimental section and which, in short, consists in measuring the mucoadhesive ability of a sample by measuring its ability to bind to mucin disposed on an inclined plane, allowing the moving away by sliding of the sample lacking mucoadhesive ability.

The method provides the use of an inclined plane which acts as support for the biological substrate, consisting of a mucin film set up by depositing, on a plexiglas plane kept horizontal, a suspension of gastric mucin (e.g., porcine) at a certain concentration in a buffer with physiological pH.

The plane is dried to obtain a film of known surface area.

An amount of known weight of the sample to be analyzed is prepared and deposited on the measurement plane with a multichannel pipette at constant time and rate for some seconds.

The total amount is measured at each assay.

The sample which does not stop on the plane is collected and measured at the end of the run. The amount of fallen sample is measured by recording the weight variation as a function of time. As control, measurements in the absence of the mucin film on the inclined plane are also performed (measurements indicated as blank) with the same amounts of blank and under the same testing conditions.

Thus, it is possible to assess the intrinsic sliding properties of the sample, regardless of its ability to interact with the biological substrate.

The adhered % is calculated: defined as the amount of sample remained adhered to the mucin film or to the inclined plane (without mucin, blank) at the end of the measurement, normalized with respect to the amount deposited on the inclined plane.

Moreover, it is determined the percentage difference between sample amount adhered to the plane with mucin and sample amount adhered to the plane without mucin. The calculation is performed in accordance with the following equation:

$$\% \text{ difference} = (\text{adhered mucin } \% - \text{adhered blank } \%) / \text{ adhered } \% \text{ of the blank})$$

Where:

- adhered mucin % = sample percentage remained adhered to the mucin film at the end of the measurement
- adhered blank % = sample percentage at the end of the blank measurements.

The barrier effect of a compound on mucous membranes or skin may be measured in any way available to the technician in the field. For instance, the barrier effect may be measured by cell marker release inhibition assay in response to an inflammatory or irritating agent as described in detail in the experimental section below.

In short, however, an *in vitro* method for the assessment of the barrier effect of fractions of extract of interest on a cell culture can be summarized with the following steps:

a) preparing a cell culture;

b) placing the sample of interest (e.g. fraction C or D according to the invention) on a semi-permeable membrane separating it from said culture, and, subsequently or concomitantly, adding to said sample a substance (e.g., an inflammatory agent such as bacterial lipopolysaccharide LPS or an interferon; an irritating or allergenic agent like ovoalbumin, dinitrochlorobenzene, oxazolone, eugenol, penicillin G, nickel sulphate) that, when in contact with said culture, be able to induce release of a marker (e.g., histamine or -glucuronidase or a cytokine like IL-8, , IL-6 , IL1, TNF, TNF) from said culture;

c) detecting in one or more successive time instants the concentration of said marker in the experiment prepared in a) and b) and comparing the measurements obtained relative to one or more reference values, wherein the lack of increase over time of said marker in step c. denotes a barrier effect of the sample.

In particular, step c) can comprise:

detecting in one or more successive time instants the concentration of said marker in the experiments prepared in a) and b) and in a parallel control experiment in which the extract fraction is not added on the permeable membrane in b) and comparing the measurements obtained from the two experiments in the same time instants.

Step c) could also comprise:

detecting in one or more successive time instants the concentration of said marker in the experiment prepared in steps a) and b) relative to the amount of said marker measured in the same experiment before step b).

In one embodiment the method can further comprise a further step of preparing an internal control in which cultured cells are pre-treated with the substance adapted to induce marker release and the extract fraction of the invention is placed on the semi-permeable membrane in the absence of said substance, which could be added later to the fraction under examination in case said substance were to settle before said fraction.

According to one embodiment, the barrier effect is measured by IL-6 inhibition assay in response to an agent such as LPS.

The depletion in grindelia most known active principles, represented by grindelic acid and optionally by saponins, is directly measurable by grindelic acid measuring, foam test and foam index.

Grindelic acid measuring as reported in the present description is obtainable with standard processes, such as, e.g., GC-MS methods. GC-MS methodology is a standard methodology that can be used by the technician in the field in the absence of the pure reference substance. In the case of grindelic acid, in the chromatogram of the sample, the area corresponding to protected grindelic acid is compared to the total area of the peaks present in the chromatogram, thereby obtaining quantitative information on grindelic acid content useful to compare grindelic acid content in different grindelia extracts and extract fractions.

As to the foam test, starting from an initial foam index of grindelia total extract, i.e. not subjected to fractioning, which, in the case of the lyophilized extract (extraction in 60% alcohol, followed by lyophilization) is of about 714, the depletion in the abovementioned active principles is directly measurable by making the proportion between the index measured for the fraction and the index measured for the reference extract by foam test as described below.

In the present invention, the barrier effect was evaluated according to the process described below and the mucoadhesive properties were evaluated by the assays (cell adhesion and adhesion with mucin on an inclined plane) them also described below. Evidently, such properties can also be evaluated with other techniques known to a person skilled in the art.

The reference extract used has a concentration of grindelic acid of about 10% in weight of the extract, a positive foam test with a foam index equal to 714, a percentage in weight of soluble macromolecules with a molecular weight of >25000 Da equal to 16.0%, a percentage in weight of total phenols titrated as gallic acid equal to 4.305, a barrier effect measured by cell marker release inhibition assay in response to an inflammatory or irritating agent (described below) equal to about 32% of inhibition of IL-6, a mucoadhesion percentage, expressed as cell adhesion percentage calculated by the process described below, equal to about 61%, and a mucoadhesion percentage, measured by adhesion assay with mucin on an inclined plane described below, equal to about 64%.

In particular, fraction C of the invention has a positive foam test, with a foam index slightly higher than the total reference extract but with a concentration of grindelic acid ≤0.5%, markedly reduced with respect to the reference extract, resulting in a depletion in grindelic acid higher than 90% with respect to the reference extract. This fraction has a composition in soluble macromolecules with a molecular weight of >25000 Da higher than 20%, equal to about 21%, a concentration in total phenols, expressed as percentage of gallic acid, of from 4 to 6 % (e.g., 4.68%) a barrier effect measured by cell marker release inhibition assay in response to an inflammatory or irritating agent equal to about 73% of inhibition of IL-6, a mucoadhesion expressed as percentage of cell adhesion, calculated with the process described

below, equal to about 69%. Therefore, fraction C is depleted of about the 85% in the active principle grindelic acid with respect to the reference grindelia extract, represented by the extract lyophilisate in 60% ethanol, whereas it is enriched in soluble macromolecules with a molecular weight of >25000 Da of about 1.3-fold, in other words contains about one twentieth of grindelic acid with respect to the reference extract, about 1.1-fold of saponins and about 1.3-fold of soluble macromolecules with a molecular weight of >25000 Da.

Fraction D of the invention has a positive foam test with a foam index ≤250 (therefore, about 35% with respect to the reference extract meant as 100%) with a substantial reduction in saponins), a percentage of grindelic acid ≤ 0.2, therefore a depletion in grindelic acid higher than 95% with respect to the reference extract; a composition in soluble macromolecules with a molecular weight of >25000 Da equal to about 5.5%, a concentration in total phenols expressed as gallic acid <4%, (e.g., equal to about 3.4%), a barrier effect measured by cell marker release inhibition assay in response to an inflammatory or irritating agent equal to about 43% of inhibition of IL-6, and a mucoadhesion percentage of >70%. According to the present invention, in fact, fraction D has a mucoadhesion percentage, expressed as cell adhesion percentage calculated with the process described below, equal to about 73%, and a mucoadhesion percentage, measured by mucoadhesion assay on an inclined plane described below, equal to about 86%. Fraction D is depleted of 94% in grindelic acid and of about 65% in saponins, and of about 65% in soluble macromolecules with respect to the reference grindelia extract consisting of the lyophilisate of the reference extract (in 60% ethanol); in other words, it contains about one fiftieth of grindelic acid with respect to the reference extract, about one third of saponins and about one third of soluble macromolecules with a molecular weight of >25000 Da.

The above-reported data demonstrate that those are not mere dilutions of the starting extract, but that the above-described processes enable to vary the relative concentrations of different components the one with respect to the other ones, therefore providing extract fractions with different features and properties. According to the above-described features of fractions C and D, the fractions of the invention will be depleted in grindelic acid of at least 80%, e.g. at least 81, 82, 83, 84, 85%, up to over 90% and, in a preferred embodiment, in saponins of at least 60%. Therefore, the fractions of the invention or mixtures thereof could be depleted in grindelic acid of from 95% to 98% and in saponins of about 60% with respect to a reference grindelia lyophilized extract as indicated above, which has not undergone fractioning.

With respect to the reference extract, which has a positive foam test with a foam index of 714, a composition in soluble macromolecules with a molecular weight of >25000 Da equal to about 16%, a concentration in total phenols expressed as gallic acid ≥4% (of from 4 to 6%), a barrier effect measured by cell marker release inhibition assay in response to an inflammatory or irritating agent equal to about 32%, a cell adhesion percentage, calculated with the process described below, equal to about 64% and a mucoadhesion percentage, measured by mucoadhesion assay on an inclined plane, described below, equal to about 64%, evidently the fractions according to the invention improve the mucoadhesive and barrier abilities of the reference extract, whereas the contents of grindelic acid, and optionally of saponins, are reduced.

The fractions selected in the present invention, in fact, exhibit a higher barrier effect and also a higher mucoadhesion with respect to the reference extract.

The Inventors of the present invention have selected the above-mentioned fractions with respect to other fractions obtainable with the process as described in the present document, as they were the only ones exhibiting sufficient reductions in grindelic acid and optionally in saponins, and having the desired mucoadhesive and/or barrier effect abilities (i.e., better than the reference extract).

As is evident from Table 2 below, many of the fractions obtained, in fact, besides exhibiting an enrichment in grindelia active principles, and therefore having a marked pharmacological effect, in some cases exhibited no barrier effect.

Therefore, evidently it is difficult to foresee which fractions of a plant extract be capable of exhibiting a barrier and/or mucoadhesion effect.

The fractions of the invention, represented by fractions C and/or D or by mixtures thereof, are therefore useful for their mucoadhesion and/or barrier effect properties in all those cases in which a protection of skin or mucous membranes is needed or desirable, which may be cases wherein a drug which attacks the mucous membranes or the skin has to be administered, therefore in order to prevent the damaging action of the drug, or in those cases in which the protection of a partially compromised skin or mucous membrane is preferable or advisable so as to enable a better and quicker healing thereof, defending it from further aggressions, or in those cases in which an individual has a chronic disorder in which skin or mucous membranes undergo irritations or alterations whereby a barrier effect can prevent or limit damages on the skin or the mucous membrane, or again in those cases in which it is desired to carry particular substances in a preferential way and with a prolonged duration on the mucous membranes.

By indicating the direct effect of protection of the mucous membranes, or of cooperating with the protection of the mucous membranes, it is meant a mechanical protection, given by the mucoadhesive abilities of the fraction itself, optionally in combination with a pharmacological protection related to added active principles (therefore, not derived from grindelia) that are preferentially carried to the mucous membranes when mixed to the fraction of the invention

thanks to the mucoadhesive abilities thereof. In the present description it is understood that said mucous membranes can be the oral mucosa, gastric mucosa, intestinal mucosa, the nasal mucosa, the vaginal mucosa, the uterine mucosa, the rectal mucosa.

Fractions C and D could be used alone or mixed with each other and could be optionally further mixed (alone or in combination) with extract of other plants and/or active principles of other plants in order to boost the mechanical activity, as defined above, of the resulting mixtures.

According to the invention, the fractions could be obtained by the process described hereinafter.

Process for the preparation of fractions of grindelia extracts depleted in grindelic acid comprising the following steps:

a. preparing a hydroalcoholic grindelia tops extract and subjecting said extract to centrifugation and/or decantation, obtaining a clarified alcoholic extract;
b. obtaining from the clarified alcoholic extract prepared in a. a concentrated aqueous extract
c. decanting and/or centrifuging and filtering said aqueous extract, collecting a water-soluble fraction C wherein said fraction is characterised by a grindelic acid concentration of ≤ 0.5%.

The fraction C obtainable by the above-described step c. exhibits quite interesting features reported in Table 1 below, i.e.:

grindelic acid concentration of ≤ 0,5%, barrier effect (BE) measured with the test as reported below

BE = % reduction in release of cytokine IL-6 = 100 - [(pg/µL cytokines released from sample/pg/µL cytokines released from C +) x 100] equal to about 73% of IL-6 inhibition, mucoadhesion percentage by adhesion test reported below equal to about 69%.

Finally, the process as described above could further comprise the following step:

d. subjecting to membrane ultrafiltration the fraction obtained in c. and collecting a fraction D, corresponding to the permeate obtained by said ultrafiltration, characterised by a foam index lower than 250 and a concentration of grindelic acid of ≤ 0.2%.

The fraction D obtainable by the above-described step d. exhibits very interesting features reported in Table 1 below, i.e.:

≥ 64% depletion in active principles saponins, >90% depletion in grindelic acid, barrier effect measured with the test as reported below

BE = % reduction in release of cytokine IL-6 = 100 - [(pg/µL cytokines released from sample/pg/µL cytokines released from C +) x 100] equal to about 43% of IL-6 inhibition, mucoadhesion percentage by adhesion assay, measured by the cell adhesion assay reported below, equal to about 73%.

The process according to the invention could then be used to produce one or both of the fractions of interest described herein, C and D.

In Table 2 below, chemico-physical features of the different fractions of grindelia obtained from the reference extract as defined and from the reference extract itself are compared.

Fraction A corresponds to the precipitate obtained from mixing of the first extract in 96% ethanol with the successive aqueous extract, in a 50:50 ratio or in alternative percentage so as to keep the alcoholic grade of the resulting mixture comprised between 40° and 60°.

Fraction B corresponds to the water-insoluble precipitate obtained by alcohol evaporation of the alcoholic extract deprived of fraction A.

Fraction C corresponds to the fraction obtained with the process of the invention.

Fraction D corresponds to the retentate fraction obtained by subjecting the water-soluble fraction obtained in c. to a step on filter with a cutoff of 1000 Da.

Fraction E corresponds to the eluted fraction obtained by subjecting the water-soluble fraction obtained in c. to a step on filter with a cutoff of 1000 Da.

Fraction F corresponds to the adsorbed fraction obtained by subjecting the water-soluble fraction obtained in c. to a step on high-porosity adsorbing styrene and DVB copolymer resin.

Fraction G corresponds to the eluted fraction obtained by subjecting the water-soluble fraction obtained in c. to a step on high-porosity adsorbing styrene and DVB copolymer resin.

## Table 2

Comparison among the different fractions analyzed

| Fraction | Grindelic acid (GC MS TMS method ) TIC - area % | Foam index determination | Hydrosoluble macromolecules with MW >25000 Da (%)* | Total phenols as gallic acid % | Barrier effect assay NET BARRIER EFFECT: % of inhibition IL-6 of the BA - % of inhibition of IL-6 of the IC | Adhesion assay with mucin on inclined plane -% | Cell adhesion assay-% |
|---|---|---|---|---|---|---|---|
| Reference Grindelia LE, SO207EL0B | 10 | 714 | 16.0 | 4.305 | 32 | 61 | 64 |
| GRINDELIA, 1st PRECIPITATED FRACTION (Fraction A) | 10 | < 100 | 25.4 | 1.141 | 45 | n.d. | 74 |
| GRINDELIA, 2nd PRECIPITATED FRACTION (Fraction A) | 20 | < 100 | 0.6 | 0.978 | 24 | 45 | 22 |
| GRINDELIA, WATER-SOLUBLE FRACTION C | 0.5 | 833 | 21.0 | 4.688 | 73 | n.d. | 69 |
| **GRINDELIA, FRACTION D PERMEATE, GE<1000 DA, LYOPHILIZED** | **0.2** | **≤250** | **5.5** | **3.430** | **43** | **86** | **73** |
| GRINDELIA, RETENTATE GE, >1000 DA, LYOPHILIZED (Fraction E) | 0.5 | 500 | 27.5 | 5.276 | <10 | 25 | n.d. |

| | | | | | | | |
|---|---|---|---|---|---|---|---|
| GRINDELIA, ADSORBED BY RESIN (OR RETENTATE BY RESIN) (Fraction F) | 1 | 625 | 19.4 | 6.703 | Detached cells | 61 | 45 |
| GRINDELIA, ELUATE (OR NON-ADSORBED BY RESIN) (Fraction G) | 0.01 | < 100 | 15.0 | 1.025 | <10 | 18 | 0 |

The table above shows how only some fractions exhibit a reduced presence of grindelic acid, and optionally of saponins, and improved mucoadhesive and barrier effect properties.
"N.d." stands for not determined (in the sense of not analyzed).

The process described herein could be carried out starting from grindelia tops, both fresh and dried, or alternatively from fluid extract (like, e.g., hydroalcoholic extracts) or dry grindelia extracts.

The dry extracts could be resuspended/resolubilized by means of hydroalcoholic solutions, e.g. by using a solid/solvent ratio of 1:5, or 1:7 or 1:8 or 1:10, putting under stirring the mass for a time ranging from 4 to 8 hours.

Step a. of the process:

a. preparing a hydroalcoholic grindelia tops extract and subjecting said extract to centrifugation and/or decantation, obtaining a clarified alcoholic extract. According to the invention, the above-described process can be carried out by performing at step a. at least two, at least three, or at least four steps in ethanol at decreasing concentrations, wherein the decreasing concentrations of ethanol are from about 96% ethanol to about 0% ethanol.

For instance, step a. of the process can be carried out by performing a step in about 85% ethanol, a step in about 50% ethanol, and a step in about 20% ethanol.

Or, step a. of the process could be carried out by performing a step in about 85% ethanol, a step in about 50% ethanol, a step in about 20% ethanol and a step in about 5% ethanol.

The extracts obtained as described above are gathered to form a hydroalcoholic extract. These can be gathered, e.g., in equal mutual ratios, such as 50:50 in case of two steps in ethanol, or such as 33.3:33.3:33.,3 in case of three steps in ethanol or 25:25:25:25 in case of four steps in ethanol. Evidently, the extracts obtained by performing the various steps in ethanol described above could be gathered into different ratios according to the general knowledge of the technician in the field. In any case, the alcoholic grade of the resulting mixture will be of from 40° to 60°, preferably from 45° and 55° alcoholic grades.

The extract thus obtained could be centrifuged or subjected to static decantation so as to obtain a precipitated fraction and a clarified alcoholic extract.

Decantation could be performed, e.g., 1 to 10 hours, and could be followed or replaced by one or more centrifugations to about 3000-4000 (e.g., about 3500) rpm for a period comprised between 1 and 10 minutes, e.g. about 5 minutes.

As already indicated above, the clarified hydroalcoholic extract obtained in step a. will be used to prepare a concentrated aqueous extract at step b.
In b. the hydroalcoholic extract decanted and/or centrifuged in a. is subjected to alcohol evaporation, thereby constituting a concentrated aqueous extract.
or c:
In step c., decanting and/or centrifuging and filtering said aqueous extract, collecting a water-soluble fraction C characterised by a concentration of grindelic acid ≤0.5%.

The supernatant obtained after decantation and/or centrifugation as described at step a. is then collected and subjected to alcohol evaporation by standard techniques, like, e.g., by use of a thin film distillation system, or a batch concentration system according to standard protocols commonly used by the technician in the field.

The result of this evaporation is a concentrated aqueous extract that, in step c. of the process, is subjected to decantation and/or centrifugation as also described in the foregoing, and finally filtered to obtain fraction C (clarified

aqueous concentrate) depleted in grindelic acid (grindelic acid concentration of ≤ 0.5%).

The concentrated aqueous extract obtained in b. may be subjected to decantation for a period comprised between 1 and 10 hours. The supernatant could be recovered and subjected to filtration.

The concentrated aqueous extract could also be centrifuged alternatively to or after the decantation, in order to collect fraction C as described above in the form of a concentrated clarified aqueous extract.

Finally, the concentrated aqueous extract is subjected to filtration to eliminate the traces of the second precipitate, thereby obtaining fraction C as a clarified solution. The filtration could be performed, e.g., on a panel filter having a cutoff of from 0.5 to 50 micrometers in a single steps or in plural steps, like, e.g. a cutoff of 0.5 micrometers, 15 micrometers, or a cutoff of 30 micrometers or even higher.

Fraction C thus obtained could be used as is or subjected to a lyophilization process to provide a lyophilized fraction useful for formulations having therapeutic employ for internal and/or external use.

In a specific embodiment, the process of the invention can be summarized as follows:

Step a. extraction at about 40°C with 96% ethanol + extraction at about 50°C with 15-5% ethanol > 1:1 mixing of the two extracts, or anyhow until the obtainment of a resulting alcoholic extract with a resulting alcoholic grade of 45°/55°,

Step b. obtainment of a concentrated aqueous fraction from the extract prepared in a., subjecting the alcoholic extract purified at about 45%-50% obtained in a. to alcohol evaporation, concentrating it about 5- to 10-fold, and

Step c. precipitate separation > obtainment of the fraction enriched in protective substances, depleted in active principles.

The process of the invention could also comprise a further step d. enabling saponins reduction jointly with a further improvement of the mucoadhesive properties of the obtained fraction with respect to fraction C and with respect to the reference extract.

In step d., fraction C. is subjected to membrane ultrafiltration and a fraction D is collected, corresponding to the permeate obtained by said ultrafiltration, characterised by a foam index of < 250 and a concentration of grindelic acid ≤ 0.2%.

In this further step, fraction C is subjected to one or more steps of ultrafiltration on membranes with a molecular weight cutoff (NMCWO) of from 500 to 50,000 daltons, e.g. of 10,000 daltons, 5,000 daltons, 2,500 daltons, 1,000 daltons. The permeate fraction thus obtained has a saponins value abated of about 60% and a grindelic acid value abated of over 90%, and is enriched in substances having a protective action and can be used as is or subjected to a lyophilization process to provide a lyophilized fraction useful for formulations having therapeutic employ for internal and/or external use.

Fractions C and D, reported in Table 2 above, are obtainable with the above-described processes.

Fractions C and D could also be suitably mixed.

The process according to the present invention, wherein the order of the steps as described above enables therefore to obtain grindelia fractions as described above, depleted in grindelic acid, optionally in saponins, and surprisingly rich in substances having a protective action.

The invention also relates to the preparation of mixtures of said fractions C and/or D with one or more natural extracts and/or active principles deriving from sources different from grindelia, such mixtures, as well as the preparation of pharmaceutical compositions containing one or more of said fractions in combination with other natural substances and/or active principles deriving from sources different from grindelia and the use of such fractions alone or mixed between them or with other components, for the protection of the mucous membranes and of the skin, or to cooperate with such protection or as carriers capable of retaining desired substances (deriving from sources different from grindelia) on the mucous membranes by virtue of their effective mucoadhesive properties.

The invention therefore relates to fractions of grindelia extracts and their uses for the protection of the mucous membranes or of the skin or as adjuvants of said protection or as excipients with good mucoadhesive properties, substantially free from the effects ascribable to commonly used grindelia active principles, i.e., grindelic acid and saponins.

The present invention also relates to a composition comprising the above-described fractions C and/or D, useful in the protection of the skin or mucous membranes wherein said fraction comprises a concentration of grindelic acid not higher than that of said fractions; in particular, said composition comprises, as grindelic acid, only the grindelic acid present in said fractions.

Said protection, of course with reference both to the fractions C and/or D per se and to the composition, could be a preventive protection or a protection of partially compromised skin or mucous membranes.

For instance, the composition or one or both of the fractions can, by virtue of their barrier effect, facilitate the repair of damaged skin tissue, with entailed restoration of a healthy and elastic skin.

The skin lesions according to the present invention are, e.g., lesions that may involve also tissue underlying the skin and in which no open wounds are present.

By skin lesions not involving the presence of open wounds, according to the present description, are meant those lesions in which the superficial layer of the skin, and the underlying layers, though not being wounded, are particularly fragile, irritated and damaged.

Non-limiting examples of this type of lesions are represented by first-degree burns, first-degree decubitus lesions, pressure lesions, newly-cicatrized rashes, wounds or burns, irritations, erhytemas.

In another embodiment the fractions of the invention could be used in addition to cicatrizing active principles not derived from grindelia, to cooperate with the healing of open wounds.

The composition or the fractions C and/or D of the invention could then be used for the treatment or the prevention of skin lesions not involving the presence of open wounds, or in the prevention or slowing down of worsenings of the same, or, in association with cicatrizing, antibacterial drugs, etc, to cooperate with the treatment of open wounds or lesions.

The compositions according to the invention could advantageously comprise further component not derived from grindelia, e.g. of plant origin, having, e.g., emollient, digestive, prokinetiks, cholagogue, carminative, prebiotic, relaxing, excipient, preservative, humectant properties. In one embodiment said substances are not extracted from grindelia.

When the composition or the fractions C and/or D of the invention are used for the protection of the skin, the application thereof will be topical. The embodiments of the composition for topical use are reported hereinafter in the description.

The composition according to the invention could be made, e.g., as oil-in-water emulsion, water-in-oil emulsion, oil-in-gel emulsion or gel-in-oil emulsion, multiple emulsions, sprays, and anhydrous formulations (pomade, gel, paste, cream, ointment) and will comprise one or more excipients suitable for the making of the desired end form.

Such excipients could be, e.g., emulsifying agents (cetearyl alcohol, cetearyl glucoside, hydrogenated castor oil), rheological additives, antioxidants (like, e.g., vitamins, tocopherols or other antioxidants known in the field).

The emulsifying agent could be a surfactant, which by lowering the interfacial tension decreases the free energy of the system; alternatively, also non-surfactant substances can be used, such as gum arabic, gelatine, hydrophilic colloids or finely subdivided powders (e.g., talc). In one embodiment, the excipients could be present at an overall concentration in weight comprised between 3 and 8%, such concentration being of course indicative, taking into account that anyhow the technician in the field will know how to adapt the concentration of the necessary excipients according to the embodiment he/she intends to prepare without addition of inventive activity.

Moreover, the composition could comprise perfuming and/or colouring agents making its odour pleasant, like, e.g., one or more essential oils like, e.g., lavender essential oil, melaleuca, lemon, mint, orange essential oil, and/or colouring agents enabling, e.g., to easily recognize the zones of application of the composition, the colouring being, e.g., temporary, so as not to interfere with other, subsequent applications of the composition.

In one embodiment, said colouring and/or perfuming agents are comprised at an overall concentration in weight between 0.001 and 3%.

By "overall concentration in weight" it is meant the concentration in weight in the composition of the sum of the various excipients, or the concentration in weight in the composition of the sum of the various perfuming and/or colouring agents present in the composition.

As to the use of the composition in the protection of the skin, the invention also relates to medical devices like, e.g., medicated plasters, medicated gauzes, medicated bandages, medicated tissues, medicated tampons, medicated diapers (pads), i.e. plasters, gauzes, bandages, tissues, tampons or diapers (pads) which comprise, or be at least partly covered by, or be at least partly soaked with the described composition of the invention in the most appropriate form.

The gauzes could be made as fatty gauzes, and so the bandages and the plasters, by using the composition made in the form, e.g., of ointment, paste or cream. The tissues could be soaked with the composition in the form of emulsion of oil with water or gel, and the diapers (pads) or the tampons could be made by inserting the composition in the suitable layers known in the field for the insertion of protective or anti-irritating compositions.

Such products, like, e.g., diapers, tampons (commonly called "sanitary napkins") for women and pads for adult incontinence, are commonly used, e.g. in the babyhood/childhood-related field, in the feminine field and in the geriatric field, and the technician in the field will know where to insert the composition described herein, and which embodiment thereof be the most suitable one, with no need of specific teachings and by basing him/herself solely on techniques conventional in the field.

Such devices will be applicable on the part to be treated and/or protected by way of prevention.

As already indicated above, the composition of the invention can be a composition for topical use that could be made in the form of oil-in-water emulsion, water-in-oil emulsion, multiple emulsions, sprays, and anhydrous formulations (pomade, ointment, gel, paste, cream, spray) according to techniques commonly used in the field. The formulation indicated herein as "spray" could be an anhydrous formulation or even an emulsion formulation, the form with an atomizer enabling also self-applications in zones harder to reach (like, e.g., the back), useful in all those cases in which the formulation is used, e.g., to alleviate sun rashes, erythemas, or skin irritations of various kind.

According to another embodiment, the fractions C and/or D or the composition of the invention could be used, thanks to their mucoadhesive and barrier effect, for the protection of the mucous membranes or as adjuvants of such protection.

In this case as well, such protection could be a preventive protection, e.g. in all those cases envisaging an administration of drugs having the side effect of attacking the mucous membranes, or in those patients exhibiting conditions of recurring irritation of the same. In other cases, the protection could be instead a protection for curative purposes or in order to avoid the worsening of irritated or partially compromised mucous membranes.

In these cases, the administration of the fractions C and/or D or of the composition could be topical for all those mucous membranes on which a topical administration is possible (e.g., oral, rectal, vaginal, nasal mucosa) or oral in the cases in which said membranes be, e.g., intestinal or gastric mucous membranes.

The compositions for the protection of the mucous membranes could therefore be made in the form of capsule, tablet, lozenge, granule, powder, syrup, elixir, hard gelatine, soft gelatine, suspension, emulsion, solution, suppository, cream, gel, spray, ointment, pomade, paste, oil-in-water emulsion, water-in-oil emulsion, oil-in-gel emulsion, gel-in-oil emulsion.

In case of formulations for topical use, the above indications related to the compositions for the protection of the skin could be followed, or syrups, rinses, sprays could be made, e.g., for the protection of the mucous membranes of the mouth, throat, nose.

For application on the rectal mucosa, suppositories or enemas or microenemas could be used, with the excipients known to the technician in the field for the making of such compositions.

As already mentioned, the composition of the invention could be in the form of capsule, tablet, lozenge, hard gelatine, soft gelatine, granule, powder, syrup, elixir, suspension, emulsion. For oral administration the composition could be made in the form of daily unit dosages or of fractions of daily unit dosages (e.g. 2, 3, 4, 5, 6, or more capsules, tablets, lozenges, granule or powder single-doses, or gelatins could be taken over the day, in accordance with the judgment of the treating doctor), and may contain conventional excipients including, e.g., binding agents, like gum arabic, gelatine, sorbitol, gum tragacanth, and/or polyvinylpyrrolidone; fillers, like lactose, sugar, corn starch, rice starch, calcium phosphate, sorbitol and/or glycine; tableting lubricants, like magnesium stearate, talc, polyethylenglycol and/or silica; disintegrants, like potato starch; and wetting agents like sodium laurylsulphate. The tablets can be coated according to methods well-known in the standard pharmaceutical practice.

The composition could also be made in a liquid or semi-liquid form, as a suspension, emulsion, solution for oral administration, and could optionally contain natural aromatizing agents giving a palatable taste thereto.

The composition in the form of powder or granule could be pre-metered in suitable containers and ready for use, either by ingestion as such or to be resuspended in an appropriate liquid such as water, tea, etc.. In this case as well, the composition could contain natural aromatizing agents giving a palatable taste thereto.

Evidently, all of the above-indicated excipients could be used in a pharmaceutically acceptable grade.

In one embodiment, the composition as described herein, in any one of the above-indicated embodiments, could be in the form of pharmaceutical composition, i.e. comprise pharmaceutical-grade ingredients or it could be or be introduced into a special-purpose food (medical food) or into a medical device.

The composition according to the present description could be made in the form of pharmaceutical composition or of medical device according to any one of the classes described in Directive 93/42/EEC on medical devices (comprising also substances and not only "devices" in the mechanical sense of the term), or in the form of medical food, food supplement, or in any form according to the regulatory provisions of the Country in which said composition will be produced.

The medical device or the medical food could also contain as ingredients other components, comprising e.g. combinations of vitamins, mineral salts and other substances directed at diet supplementing.

The fractions C and/or D as described herein or the composition of the invention are/is therefore useful for its mucoadhesion and barrier effect properties in all those cases in which the protection of skin or mucous membranes is needed or desirable, or where it be needed or desirable to cooperate with such a protection: those may be cases in which a drug that attacks mucous membranes or skin has to be administered, therefore in order to prevent or limit the damaging action of the drug, or in those cases in which the protection or the cooperation with the protection of a partially compromised skin or mucous membrane is preferable or desirable so as to enable a better and quicker healing thereof, defending it from further aggressions, or in those cases in which an individual has a chronic disorder in which skin or mucous membranes sustain irritations or alterations, therefore a barrier effect can prevent or limit damages on the skin or the mucous membrane.

The invention also relates to a process for the treatment or for the prevention of the onset or the worsening of skin lesions not involving the presence of open wounds, wherein such process comprises one or more applications of the composition of the invention or of the medical device comprising it once or more per day on the concerned part.

The application of the composition, for instance, could be repeated whenever needed (e.g. at each change of pad in case of prevention of incontinence-related rashes) or once, twice, thrice, four or more times per day in general.

The invention also relates to a process for the preparation of a composition according to any one of the embodiments described above, wherein the above-described fractions C and/or D are mixed with at least one among excipients and/or substances of natural and/or plant origin having emollient, digestive, prokinetics, cholagogue, carminative, prebiotic, relaxing, excipient, preservative, humectant properties as described above. Such substances, of course, will not consist in grindelic acid and/or saponins.

Among these, e.g., could be used one or more of: gentian root extract, boldo leaves extract, milk thistle fruits extract, artichoke leaves extract, taraxacum root extract, anise fruit extract, rosemary leaves extract, mint leaves extract, marjoram leaves extract, cumin fruit extract, coriander fruit extract, ginger root extract, fennel fruit extract, caraway fruit extract, charcoal, inulin, plantain leaves extract, helichrysum flowering top extract, thymus flowering top extract , aloe vera gel, althea root extract, arnica flower extract, jojoba oil, honey.

Object of the present invention is also a method for the protective (preventive or curative) treatment of the skin or mucous membranes providing the administration of the fractions C and/or D or the composition of the present invention to a patient in need thereof. Such administration could also be concomitantly with the administration of other drugs.

Therefore, by "protective treatment" for the purposes of present invention, it is meant a treatment of damaged, irritated or irritable (e.g., if the use of an irritating drug is envisaged) skin or mucous membranes wherein the fraction or the composition of the invention form a protective film thanks to the mucoadhesive power of the fraction of the invention, and thereby enable a healing of the damaged, wounded and/or irritated mucous membranes, or prevent damage thereto which may be caused by therapeutic treatments that must be administered to the patient, by administering the fraction or composition of the invention before the drug.

Alternatively, the fraction or composition could be administered together with active principles that is desirable to retain longer on the mucous membrane in order to prolong or make more effective the therapeutic effect thereof.

The strong depletion in pharmacologically active grindelia principles in the fractions or in the composition according to the invention, along with an improvement of the mucoadhesive and barrier activities, makes such products particularly suitable to administration concomitantly with other drugs, as a side effect of interaction between one or both of the fractions C and/or D, or between the composition of the invention and the drug co-administered or concomitantly administered is markedly unlikely.

A non-limiting example of the method of treatment and/or of prevention of the skin or mucous membranes, could comprise the administration of a daily dosage, subdivided into a single dose or plural doses, described of the mixture or the composition according to the present description, for a period of time of between one and six weeks, e.g. of between three and six weeks or even for a period of time higher than six weeks, in accordance with the judgment of the treating doctor.

Such administration could precede the administration of the drug even for a prolonged period, so as to optimize the health state of the skin or of the mucous membrane to be treated.

The treating doctor will know how to establish both the most appropriate dosage and the administration times also on the basis of the patient's health state, weight, sex and age.

One of the substantial differences between the structure of the skin and that of the mucous membranes is represented by the absence, in the mucous membranes, of a selective barrier such as the corneous layer. Oral mucous membranes contact with noxious or irritating substances present in the environment (pollutants, pathogenic microorganisms, etc.) can therefore cause a high penetration of said substances both inside the mucous membranes and in the related airways (bronchi, lungs, etc.) causing inflammatory and/or allergic pathologies.

The protective effect of the fractions C and/or D of the present invention was assessed by mucoadhesion assays and by barrier effect assay.

The former aim at evaluating the mucoadhesivity of the product under examination in order to establish whether such product has the ability to adhere to the mucous membranes and consequently exert a protective action.

Two simple models for evaluating mucoadhesivity of the product of interest were used, providing the use of intestinal mucosal cells. In particular, mucoadhesivity was evaluated by assay of adhesion to mucin on an inclined plane and by determining the percentage of inhibition of the lectin/glycoprotein bond; in this latter case evaluation was carried out by using a commercial intestinal cell line, HT-29.

For evaluation of the barrier effect an *in vitro* model is available enabling to evaluate the hindrance of the sample to transit of LPS, accountable, in the model, for the freeing of mediators such as interleukin 6 (IL-6) measured semi-quantitatively by ELISA assay.

<u>**Evaluation of the percentage of inhibition of the lectin/glycoprotein bond**</u>

Mucoadhesivity is determined by evaluation of the percentage of inhibition of the lectin/glycoprotein bond. In this model, for instance, buccal, gastric, intestinal or vaginal cells may be used. The cells were initially treated with biotinylated lectin (Con-A), having high affinity for the glucoside and mannoside residues present in the glycoproteins of the membrane. The sites of the glycoproteins of the mucosal membranes will thus be occupied with the biotinylated

lectin. The presence of biotin (vitamin H) in the lectin is indispensable for the next stage. The cells already treated with biotinylated lectin are charged with streptavidin peroxidase, making it possible, thanks to the high affinity between biotin and streptavidin, to form the protein-glucose-lectin-biotin-streptavidin peroxidase complex. At this point, the cells are washed and the protein-glucose-lectin-biotin-streptavidin peroxidase complex is quantified, thanks to the presence of peroxidase, by means of a reaction of oxidation of the ortho-phenylenediamine.

The protein/glucose/lectin/biotin/streptavidin peroxidase complex catalyses the polymerization reaction:

$$\text{O-phenylenediamine} \xrightarrow[\text{Strep.perox}]{H_2O_2} \text{2,3-diaminophenazine (yellow).}$$

The intensity of the yellow/orange coloration of the solution (measured using a spectrophotometer with =450 nm) is proportional to the quantity of glycoprotein/lectin bonds and therefore to the quantity of available sites (glycoproteins) for mucoadhesion. The absorbency value thus determined constitutes the "control".

In the mucoadhesion assay reported below, the gastrointestinal cells are treated for about 15 minutes at 30°C with the product under examination before the treatment with lectin. In the presence of mucoadhesive products, said products will inhibit lectin bonding, decreasing, proportionally to their mucoadhesion ability, the signal strength in the sample compared to the control as described above.

The percentage of mucoadhesion of the product (%MA) could be determined as

$$\%MA = (1 - \text{abs sample/abs control}) \times 100$$

**Barrier effect assay**

The barrier effect assay is a biological-type *in vitro* assay employed for assessing the ability of finished products and/or raw materials to protect, through the formation of a thin "insulating" layer, mucous membranes and skin from contact with environmental contaminants (dust, pollens, microorganisms, etc.)

The assay was developed to simulate *in vitro* the action exerted by products that are applied on skin and/or mucous membranes with the aim of creating a protective film against external aggressors.

The model takes advantage of the principle whereby cells subjected to contact with an inflammatory agent produce and secrete pro-inflammatory mediators (cytokines) in the extracellular environment in an amount related to the degree of inflammation caused; within a certain range, a direct proportionality exists between concentration and times of exposure to the inflammatory agent and amounts of cytokines released.

The experimental model adopted provides two chambers physically separated by a semi-permeable membrane (0.4μm pores). Cells are seeded in the lower chamber whereas the upper chamber accommodates the inflammatory agent; on the semi-permeable membrane separating the two chambers a thin film of the sample under analysis is stratified to highlight, if present, a barrier effect to the free transit of the inflammatory agent.

The semi-permeable membrane allows passage of the inflammatory agent in the lower chamber and constitutes the support on which the sample to be assayed is stratified. Depending on the "insulating" ability of the sample, a decrease of LPS migration from the upper chamber to the bottom one will be had (therefore a lesser stimulation of cells to cytokine production).

EXAMPLES

**Example 1**

**Preparation of fractions C and D**

Grindelia tops were fragmented and subjected to two steps of extraction in ethanol at decreasing concentrations; performing a step in 96% ethanol% at about 40°C, and a step in 5% ethanol at about 50°C.

The alcoholic extracts obtained as described were gathered in a 50:50 ratio, or anyhow until obtainment of an alcoholic mixture having an alcoholic grade of 45-55°, were subjected to decantation; the supernatant was recovered and subjected to centrifugation for a time of 1-5 minutes at a rotation rate equal to 3,500 rpm. Thus, an efficient separation between supernatant and precipitate is attained, and both are recovered. The supernatant was concentrated by ethanol evaporation with use of a thin film distillation system according to the standard protocol, providing the feeding of the extract

to be concentrated at a rate of about 500 l/h; operation was by setting a residual vacuum of 06-0.8 bars and the fluid heating the evaporation walls was set at 140°C, thereby obtaining, after ethanol elimination, a concentrated aqueous extract and a second precipitated fraction (also this precipitate is separated and recovered to be used for aims differing from those of the present patent). The concentration ratio in this phase is of from 3:1 to 10:1.
The aqueous extract thus obtained was filtered on a panel filter with a cutoff of 25 micrometers and consecutively on a panel filter with a cutoff of 0.5 micrometers, obtaining fraction C.
The fraction C thus obtained comprised grindelic acid at concentrations reduced over 90%, whereas the saponins had a concentration similar to the initial extract. The fraction C was subjected to a step of ultrafiltration on membrane with a cutoff of 1,000 daltons, yielding a permeate fraction D with grindelic acid reduced over the 95% and saponins reduced over the 60%.

Fraction E was obtained by collecting the retentate derived from the passage of the water-soluble fraction obtained in c. on an ultrafiltration membrane with a cutoff of 1,000 Da.

<u>Fractions F and G were obtained</u> by subjecting the water-soluble fraction obtained in c. to a passage on high-porosity adsorbing styrene and DVB copolymer resin and collecting the resin-adsorbed fraction and that eluted therefrom, respectively.

**Example 2**

**Assessment of mucoadhesive ability of the extract of the invention by evaluation of the percentage of <u>inhibition of the lectin/glycoprotein bonding</u>**

The method described hereinafter derives from the optimisation of previous experimental protocols and scientific works on the topic (1-18), the mucoadhesivity of products intended for treatment of mucous membranes can be determined by evaluation of the percentage of inhibition of the lectin/glycoprotein bond. Mucosal cells of different origin (buccal, vaginal, gastrointestinal, etc.) can be used in this model. The assessment of mucoadhesive ability of <u>Grindelia fractions</u> was carried out by using a HT-29 cell line. The intestinal cells of the HT-29 line were selected as a model of epithelial cells, since they have morphological and biochemical characteristics typical of the absorbing enterocyte present in the gastrointestinal tract and have been widely used to study the functionality of the gastrointestinal cells and also to assess the transport and/or interaction of drugs through the gastrointestinal membrane (19-21). The bonding of the substance was assessed depending on the percentage of lectin-glycoprotein inhibition. Lectin is a protein contained in some leguminosae (*Canavalia ensiformis*), having a high affinity for the glucoside and mannoside residues present in the glycoproteins of the membrane. Lectin was artificially bonded to biotin (Con-A), which was able to bond to the streptavidin peroxidase that was added to the cellular suspension. Thus, the sites of the glycoproteins of the mucosal membranes would be occupied with the biotinylated lectin. After a washing, o-phenylenediamine (o-pd) was added to the cell suspension in the presence of hydrogen peroxide. If in the reaction environment streptavidin peroxidase is present, o-pd is oxidised, with development of a yellow colour. The reaction was stopped after 2 minutes with$H_2SO_4$ 1N. The intensity of the yellow/orange colouration of the solution (measured using a spectrophotometer with =492 nm) is proportional to the quantity of bonds of lectin with the glucosi de groups on the cell membrane. In case of cells treated preliminarily with a product having mucoadhesive ability (incubation at 30°C for 15 minutes, before the treatment with lectin), the sites for bonding of lectin are occupied by the substance under examination, determining a decrease in the intensity of the end colouration, since less streptavidin peroxidase will be bonded to the biotinylated lectin. The decrease in the absorbency value will be proportional to the ability of substances under examination to "mucoadhere" to the cells.
The mucoadhesive ability is expressed as a **percentage of inhibition of glycoprotein-lectin bonding,** or better as percentage of mucoadhesion of the product according to the equation:

***Percentage of mucoadhesion of the product = (1 – abs sample/abs control) x 100***

<u>**Materials and methods**</u>

The HT-29 cells were kept in Mc Coy's with the addition of 10% of foetal bovine serum, 100 U/ml of penicillin and 100 g/ml of streptomycin and held at 37°C in an incubator under humidified atmosphere formed of 95% air and 5% $CO_2$. The culture medium was changed every 2-3 days. Before the experiment, the cells were trypsinized, counted in a haemocytometer, centrifuged at 1000 rpm for 10 minutes and resuspended in 2 ml of culture medium. Then, the cells (contained in a total volume of 1 ml), subdivided into as many 50ml conical-bottom test tubes as the samples were, were

treated with the solutions containing the sample or with the sole vehicle (BLANK: 5 ml of physiological solution). The solution containing the sample, contacted with the cell suspension, has an end volume of 5 ml; said volume is constituted 50:50 by physiological solution and solution containing the sample, in case of samples dissolved in hydroalcoholic solutions. In particular, the Inventors' samples were dissolved as follows: 60% hydroalcoholic solution in case of the reference extract, 20% hydroalcoholic solution in case of fraction C, physiological solution in case of fraction D. Similar responses were obtained in case of sample solubilisation in physiological solution. The conical-bottom test tubes were left under gentle stirring at 30°C for 15 minutes. The cells were then washed 3 times in TBS 0.05 M. 5 ml of TBS 0.05M containing $CaCl_2$ 1 mM and 10mg/L of biotinylated lectin were then added, for 30 minutes at 30°C under slow stirring. After 1 wash, the cellular suspension was incubated with 5 ml of TBS 0.125M containing 5mg/L of streptavidin peroxidase and left to incubate for 60 minutes at 30°C under slow stirring. The cells were then washed 1 time and lastly resuspended in 1 ml of solution of o-pd (containing 0.4 mg of o-pd in citrate buffer and 0.4 l of $H_2O_2$).
The oxidation of the o-pd produced a yellow colouration and the reaction was stopped after 2 minutes by the addition of $H_2SO_4$ 1 N. The optical density was measured at 492 nm by means of spectrophotometric reading.

The results obtained, reported in Tables 1 and 2 above, demonstrate that the assayed fractions of extract possess a high mucoadhesive ability AT LOW CONCENTRATIONS with respect to mucosal cells. In light of the results obtained, it is possible to state that FRACTIONS C and D ALONE OR IN MIXTURE, demonstrating to possess high, resistant mucoadhesivity, can play an interesting protective role on mucosal cells.
the different chemico-physical features of the fractions make necessary different solvents to carry out the assay.

**Example 3.**

**Barrier effect assay**

(For the barrier effect assay, two chambers physically separated by a semi-permeable membrane (0.4μm pores) were used. Human fibroblast cells were seeded in the lower chamber, whereas the inflammatory agent LPS (purified E. Coli lipopolysaccharide) was introduced into the upper chamber; on the semi-permeable membrane separating the two chambers a thin film of grindelia fractions as described in the present invention, and of other fractions as reported in Table 1 and in the extraction diagram depicted in Figure 1, was stratified.

Induced inflammatory response was estimated through semi-quantitative dosage of Interleukin 6 (IL6) cytokine released in the culture medium of the lower chamber: Barrier effect assessment was obtained by comparison with the positive control in which the two chambers were separated by the same type of semi-permeable membrane, free however from any barrier.

The greater the barrier effect exerted by the stratified film of substance, the lesser the inflammatory action observed in the cells present in the chamber therebelow, as the inflammatory agent in the chamber thereabove will have greater difficulty to cross the semi-permeable chamber.

As to the threshold value above which it may be said that a substance causes a barrier effect in the assay reported herein, a value equal to 15% of inhibition as compared to the control was identified by the Inventors, during the setting up of the assay, on the basis of assays performed on substances known to have a barrier effect.

Then the barrier effect of the fractions as described herein was evaluated.

The data reported below show how the fractions have a remarkable barrier effect.

The assessment assay was carried out as follows through a method developed to simulate *in vitro* the protective action of substances and formulations which, when applied to the skin and mucous membranes *in vivo,* form an "insulating" film against environmental agents.

The model takes advantage of the principle whereby cells subjected to contact with an inflammatory agent produce and secrete pro-inflammatory mediators (cytokines) in the extracellular environment in an amount related to the degree of inflammation caused. The greater the amount of inflammatory agent reaching the cells, the greater the amount of cytokines released.

The model envisages the arrangement of two chambers physically separated by a semi-permeable membrane which allows the passage of sufficiently small-sized solutes.

In the lower chamber, consisting of a well containing plates for cell cultures, HuDe cells (no. BS PRC 41, bought from the Istituto Zooprofilattico di Brescia, Italy) are grown, while the upper chamber, consisting of an insert for complex cell cultures (transwells), accommodates the inflammatory agent.

On the surface of the semi-permeable membrane of the insert separating the two chambers, before the introduction of the inflammatory agent in the upper chamber, a thin film of the sample being examined is stratified to assess any BE upon the free passage of the inflammatory agent.

As a function of the insulating capabilities of the sample, there will be had a decrease of the migration of the inflammatory agent from the upper chamber and, as a consequence, a lesser stimulation of the cells to produce cytokines. The extent of the inflammatory reaction is estimated through the semi-quantitative dosage of cytokines released in the

culture medium of the lower chamber, in particular of interleukin 6 (IL-6).

As a control a similar experiment is used in which no sample is stratified on the membrane, thus making it possible to measure the effect of the inflammatory agent without any barrier in addition to the semi-permeable membrane.

Further, an internal control is used in which the cultured cells are pre-treated with the substance adapted to induce the release of the marker and the sample is placed on the semi-permeable membrane in the absence of said substance, one or more measurements in time are thus performed of the quantity of marker in the culture medium of said internal control. In the internal control, the cells are therefore stimulated first with the inducing substance and then there has to be assessed whether the sample which may pass the membrane and go into the cells pushed by the medium above has any effect in decreasing the release of the marker not related to the barrier effect. For instance, when an inflammatory agent is used as the inducing substance, the internal control makes it possible to understand if the reduction in the concentration of cytokines in the culture medium is due to the barrier effect or if the sample that may pass into the cells pushed by the medium above has any effect in reducing the inflammatory response independently of the barrier effect.

The barrier effect (BE) is expressed as a percentage of the reduction of the release of IL-6 and is calculated through comparison with the positive control in which the two chambers are separated by the same type of semi-permeable membrane without the barrier created by the sample.

3.1 PREPARATION OF THE CELL CULTURE:

For each assayed sample, cells were seeded in the wells of a cell culture plate, one for the barrier assay (BA) and another one for the internal control at a density of 40,000 cells /ml in MEM medium supplemented with 10% bovine serum (FBS); 1 ml of cell suspension per well.

The cells were treated with the SAMPLE (CAM), with the POSITIVE CONTROL (C+) (inflammatory agent without sample), and with the NEGATIVE CONTROL (C-) (medium only) and each assay is carried out in triplicate.

The plates were incubated at 37°C, overnight (22-24 hours).

3.2 PREPARATION OF INSERTS FOR COMPLEX CELL CULTURES

Inserts for complex cell cultures (Becton Dickinson) are placed on other plates, and on each of them a fixed amount of collagen of 0.1 mg/ml is supplied. The plates were incubated at 37°C, overnight (22-24 hours).

3.3 VERIFICATION OF STATE AND LEVEL OF CELL CONFLUENCY

In order to proceed with the experiment, a confluency not lower than 95% is required.

3.4 COLLAGEN LAYER DRYING

From the two plates (BA and IC) with the inserts the collagen is removed and the insert left under the flow of the hood for the time required to let them dry completely (10÷15 minutes).

3.5 BARRIER ASSAYS (BA)

The steps described below were carried out in the culture plate for the BA.

Setting up of the sample layer in the BA:

On the sample's semi-permeable membrane 100μl of a composition comprising the fractions described in the invention were inoculated and allowed to stratify for 20 minutes while in the C+ and C- inserts nothing was added. Once the 20 minutes had elapsed, excess sample was eliminated and the membranes washed with PBS according to procedures specified by the protocol.

Addition of LPS (inflammatory agent) to BA inserts

Once the sample layer had dried, in the first three CAM inserts and in the three C+ ones, 300μl of the LPS (membrane lipopolysaccharide) solution were inoculated at the concentration of 1μg/ml while in the remaining three of the C- 300 μl of MEM medium with 5% FBS were added. The inserts were inserted in their respective wells with the cells and the plates were incubated for 1 h at 37°C and under an atmosphere enriched with 5% $CO_2$ overnight (22-24 hours).

Once completed the 1 h incubation, the inserts were removed and discarded and the plates were incubated again overnight (22-24 hours).

3.6 INTERNAL CONTROL (IC) ASSAY:

The internal control assay was carried out concomitantly with the BA.

Exposure of IC cells to LPS:

Once dried, in the first six inserts of IC, three for the sample to be analysed and three for the C+, 300μl of the LPS solution were inoculated while in the remaining three of the C- 300μl of the medium were added.
The inserts with LPS and MEM were then inserted in the wells with IC cells and all incubated for 1 h.

LPS removal and IC membrane drying:

Once completed the 1 h incubation, the inserts were removed from the wells with the cells and transferred to the empty plate, while the plate with the cells was placed in an incubator.
The LPS solution still present was removed from the inserts, the latter were subjected to a rapid wash with ultrapure sterile water and allowed to dry.

Arrangement of sample layer in the IC:

On the semi-permeable membrane of the three inserts for the sample, 100μl of a composition comprising each fraction according to the invention under examination were inoculated and allowed to stratify for 20 minutes while in the C+ and C- inserts nothing was added. Once the 20 minutes had elapsed, the excess sample was eliminated and the membranes were washed with PBS according to procedures specified by the protocol.

Addition of LPS to IC inserts

Once the inserts with the sample were ready, 300 μl of medium were added to all inserts (CAM, C+, C-). The inserts were inserted in their respective wells with the cells and the plates incubated for 1 h at 37°C.
Once completed the 1 h incubation, the inserts were removed and discarded and the plates incubated again overnight (22-24 hours).

4.7 SUPERNATANT COLLECTION AND ENZYME IMMUNOASSAY

One the 22-24 hours had elapsed, the supernatants were collected from the BA and the IC plates for performing the ELISA assay and the semi-quantitative dosage of IL-6

BARRIER EFFECT (BE) ASSESSMENT

The BE of a substance or compound is expressed as *% reduction in the release of IL-6 cytokine* by cells exposed to LPS wherein the sample has been assayed relative to the positive control (C+) wherein the cells have only been exposed to LPS.

**BE = *% reduction in release of IL-6 cytokine* = 100 - [ (pg/μL cytokines released from sample / pg/μL cytokines released from C +) x 100]**

Tables 1 and 2 above show that the fraction C and the fraction D according to the present invention creates a barrier to passage of LPS.
As may be seen from the Table, the fractions of the invention exhibit a good barrier effect, associated with an effective mucoadhesive ability, making them suitable to the uses claimed and described in the present application.

**Example 4.**

FOAM TEST

About 1 g of sample is boiled in 50 ml of distilled water under slow magnetic stirring. After the boiling process, it is filtered in a 50ml flask, using filter paper. It is cooled down and brought to volume with distilled water. 5 ml of distilled water are added to 10 ml of filtrate. The mixture is vigorously stirred for about 5 minutes, allowing formation of a foam. A positive result is indicated by the forming of a lasting and stable foam for at least 30 minutes.
Then, three drops of olive oil are added to the foam, and it is vigorously stirred for about 1 minute. A positive result is indicated by the forming of an emulsion.

**EXAMPLE 5.**

FOAM INDEX

About 1 g of finely powdered and sifted sample is weighed, and the weighed product is introduced into an Erlenmeyer flask containing 100 ml of boiling water. The whole is boiled for 30 minutes. It is cooled down and filtered on paper in a 100ml flask, then bringing it to volume with distilled water. Portions respectively of 1 ml, 2 ml, 3 ml, up to 10 ml are placed in as many 15ml Falcon test tubes and the volume of liquid in each test volume is brought to 10 ml with distilled water. The test tubes are then closed with the cap and vigorously agitated for 15 seconds in a longitudinal direction. It is left to settle for 15 minutes and foam height is measured.
The results are assessed in the following way:

- If foam height in all test tubes is lower than 1 cm, the foam index is ≤ 100; in this case, in fact, the volume of undiluted sample is used to perform the calculation (therefore, I=1000/10 ml = 100, this means that not even the undiluted sample is able to provide a lasting 1-cm foam),
- If foam height in one of the test tubes is of 1 cm, the volume of plant material in that test tube is employed to measure the index. If the test tube is the first one [test tube a, containing 1 ml of the mother solution I=1000/1 (as 1 ml of mother solution is in test tube a)=1000] or the second one [test tube b, containing 2 ml of the mother solution I=1000/2 (as 2 ml of mother solution are in test tube b)= 500], prepare likewise an intermediate solution in order to obtain more precise data [test tube c, containing 1.5 ml of the mother solution I=1000/1.5 (as 1.5 ml of mother solution are in test tube c)= 750].
- If in each test tube the foam height exceeds 1 cm, the foam index is >1000. In this case, repeat the determination using a new series of dilutions to obtain a result.

CALCULATIONS:

The calculation of the foam index is performed by using the following formula:

$$I = \frac{1000}{a}$$

Where:

a = the volume in ml of the decoction employed to prepare the dilution in the test tube in which the foam height was of 1 cm.

**Example 6.**

Titer in grindelic acid

Sample preparation: 0.25 g of sample were extracted with 5 ml of a methanol/dichloromethane 3:1 mixture for 30 minutes under ultrasounds (T°=35° C, W=70%). After centrifugation, on the pellet a second extraction is repeated under the same conditions. The solution is brought to volume on a 10ml calibrated flask. In a micro test tube, 100 microliters of sample were brought to dryness under nitrogen stream. The residue was additioned with 80 microliters of hexane, 10 microliters of pyridine, 20 microliters of BSTFA. The micro test tube with a sealing cap was placed in a heater at 60° C for 30 minutes. At the end, 5 microliters of a nerolidol solution were added. The resulting solution was ready to

be injected into GC-MS.

**Chromatographic method**

Injector: 280°C, Split ratio 15:1.
Column_HP-05MS
Injection volume: 2 microl
Internal standard for signal quality control: Nerolidol

**Chromatographic conditions.**

Carrier: Helium 1.2ml/min
Chromatographic ramp: 80°C for 2min, 10°C/min until reaching 200°C, 6°C/min until reaching 280°C.

**Acquisition method**

EI mass: 70eV.
Mode:SCAN
Masses: 50-700
The TIC (Total Ion Chromatogram) chromatogram of the fractions of interest was compared with that of the reference extract. Evaluation of grindelic acid presence was performed by calculating the percentage area of the peak with respect to the TIC area. Such methodology enabled the Inventors to have an assessment on the relative amount of the analyte, and therefore enabled them to verify that the fractioning had determined a conspicuous decrease of the grindelic acid in fractions compared with the reference grindelia extract.
Grindelic acid has a retention time equal to 24.5 minutes. Recognition was performed by study of ionization, analyzing the characteristic fragmentation signals which are: **465** (molecular ion +2TMS), **268** (molecular ion + 1TMS), **187** (fragment due to the substituted cyclo-decalene), **109.**

**Example 7.**

__**Evaluation of adhesion percentage by inclined plane with mucin.**__

Measurements are performed by an equipment comprised of a 45°-inclined plane, thermostated to 37°C; below said plane a microbalance is set, which performs measurements at regular 1-s intervals and prints recorded measurements on paper. Performed measurements are then transcribed and reprocessed on Excel spreadsheet.
The inclined plane acts as support for the biological substrate, consisting of a mucin film set up by depositing, on a plexiglas plane kept horizontal, a volume equal to 3ml of a suspension of porcine gastric mucin at an 8% concentration (w/w) in a phosphate buffer, pH 6.4.
The dispersion is then brought to dryness (optionally also at the temperature of 44°C) so as to obtain a film of known surface area equal to 33.6 $cm^2$.
An exactly weighed amount of the fraction according to the invention is dissolved in a suitable solvent, physiological solution included. The fraction is then deposited on the measurement plane with a multichannel pipette at constant time and rate during 6 seconds.
The multichannel pipette is calibrated so as to meter the solution (or dispersion) over the work plane. The total amount is measured at each assay (the weight of said volume is measured before the assay and depends on the density of the sample under analysis).
The sample, which will fall on the microbalance at the end of the run, is loaded in the top part of the inclined plane, containing the mucin film, and let slide thereon. The amount of fallen sample is measured by recording the weight variation as a function of time. The assay as made herein lasts 40 seconds, though usually after 20 seconds there is no weighing variation.
Measurements in the absence of the mucin film on the inclined plane are also performed (measurements indicated as blank) with the same amount of sample and under the same testing conditions.
Thus, it is possible to assess the intrinsic sliding properties of the sample, regardless of its ability to interact with the biological substrate.
The adhered % is calculated: defined as the amount of sample remained adhered to the mucin film or to the inclined plane (without mucin, blank) at the end of the measurement, normalized with respect to the amount deposited on the inclined plane.
Moreover, it is determined the percentage difference between sample amount adhered to the plane with mucin

and sample amount adhered to the plane without mucin. The calculation is performed in accordance with the following equation:

$$\% \text{ difference} = (\text{adhered mucin } \% - \text{adhered blank } \%)/\text{adhered } \% \text{ of the blank}$$

Where:

- adhered mucin %= sample percentage remained adhered to the mucin film at the end of the measurement
- adhered blank % = sample percentage at the inclined plane at the end of the blank measurements.

## Claims

1. A fraction of a grindelia flowering tops extract depleted in grindelic acid, **characterised by** a grindelic acid concentration of ≤0.5% and by a barrier effect measured by cell marker release inhibition assay in response to an inflammatory or irritating agent higher than or equal to 40%.

2. The fraction according to claim 1, wherein said fraction is **characterised by** a grindelic acid concentration of ≤0.2%; by a foam index of about 250 and by a concentration in weight of soluble macromolecules with a molecular weight of >25000 Da of from 4 to 7%.

3. The fraction according to claim 2, further **characterized by** a mucoadhesion percentage of >70%.

4. The fraction according to any one of claims 1 to 3 for use in a treatment for the protection of skin or mucous membrane.

5. The fraction according to claim 4 wherein said mucous membrane is the oral mucosa, gastric mucosa, intestinal mucosa, the nasal mucosa, the vaginal mucosa, the uterine mucosa, the rectal mucosa.

6. A process for the preparation of a fraction of grindelia depleted in grindelic acid comprising the following steps:

    a. preparing a hydroalcoholic extract of grindelia flowering tops and subjecting said extract to centrifugation and/or decantation, obtaining a clarified alcoholic extract,
    b. obtaining from the clarified alcoholic extract prepared in a. a concentrated aqueous extract,
    c. decanting and/or centrifuging and filtering said aqueous extract, collecting a water-soluble fraction C wherein said fraction is **characterised by** a grindelic acid concentration of ≤0.5% and by a barrier effect measured by cell marker release inhibition assay in response to an inflammatory or irritating agent higher than or equal to 40%.

7. A process according to claim 6, further comprising the step:

    d. subjecting to membrane ultrafiltration the fraction obtained in c. and collecting a fraction D, corresponding to the permeate obtained by said ultrafiltration, **characterised by** a foam index of ≤250 and a concentration of grindelic acid ≤0.2%.

8. A pharmaceutical carrier consisting of one or more fractions according to claims 1 to 6, wherein said carrier promotes the adhesion of active principles to mucous membranes.

9. A composition comprising one or more fractions according to any one of claims 1 to 6.

10. The composition according to claim 9 for use in a treatment for the protection of skin or mucous membrane.

11. The composition according to claim 10 wherein said mucous membrane is the oral mucosa, gastric mucosa, intestinal mucosa, the nasal mucosa, the vaginal mucosa, the uterine mucosa, the rectal mucosa.

12. The composition according to any one of claims 10 to 11 further comprising substances of natural and/or plant origin having emollient, digestive, prokinetics, cholagogue, carminative, prebiotic, relaxing, excipient, preservative, humectant properties.

**13.** The composition according to claim 12 wherein said substances of natural and/or plant origin are selected from one or more from: gentian root extract, boldo leaves extract, milk thistle fruits extract, artichoke leaves extract, taraxacum root extract, anise fruit or aniseed extract, rosemary leaves extract, mint leaves extract, marjoram leaves extract, cumin fruit extract, coriander fruit extract, ginger root extract, fennel fruit extract, caraway fruit extract, plantain extract, helichrysum extract, thymus extract, arnica flower extract, althea root extract, aloe vera gel, charcoal, inulin, jojoba oil.

**14.** The composition according to any one of claims 9 to 13 in the form of capsule, tablet, lozenge, granule, powder, syrup, elixir, hard gelatine, soft gelatine, suspension, emulsion, solution, suppository, cream, gel, spray, ointment, pomade, paste, oil-in-water emulsion, water-in-oil emulsion, oil-in-gel emulsion, gel-in-oil emulsion.

**15.** The composition according to any one of claims 9 to 14 wherein said composition is a pharmaceutical composition, is comprised in or consists of a medical device, is comprised in or consists of a food supplement or is comprised in a medical food.

**16.** Use of the fraction according to claims 1 to 4 or of the composition according to claims 9 to 15 for the protection or for cooperating with the protection of skin or mucous membranes from aggressive or irritating agents.

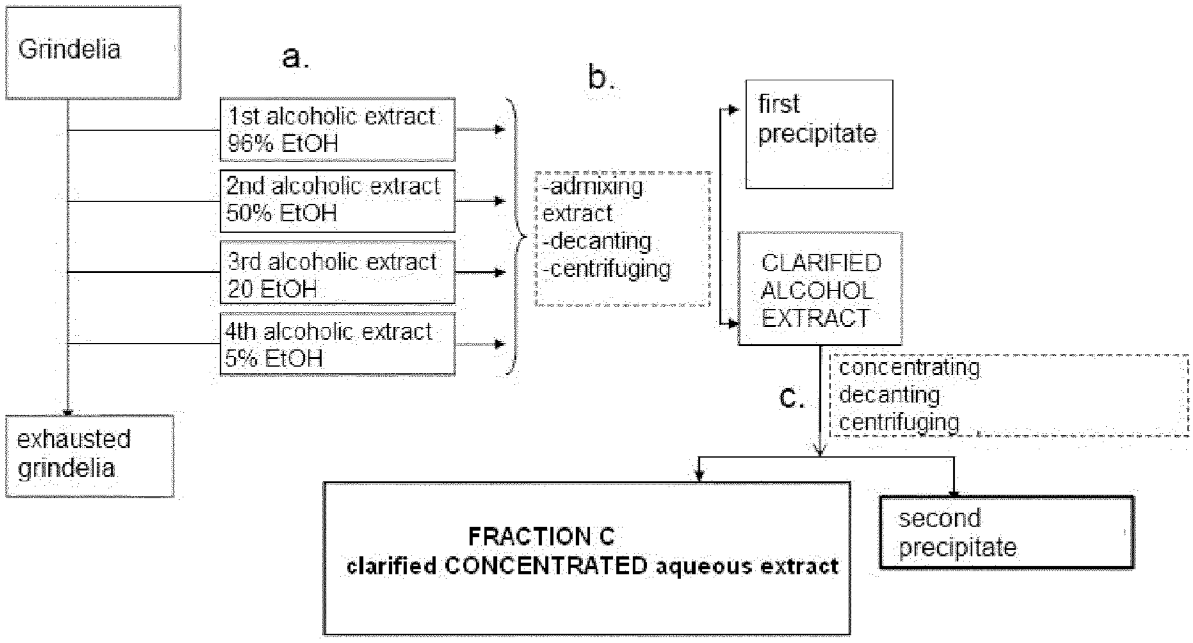
Grindelia
a.
b.
1st alcoholic extract 96% EtOH
2nd alcoholic extract 50% EtOH
3rd alcoholic extract 20 EtOH
4th alcoholic extract 5% EtOH
-admixing extract -decanting -centrifuging
first precipitate
CLARIFIED ALCOHOL EXTRACT
c.
concentrating decanting centrifuging
exhausted grindelia
FRACTION C clarified CONCENTRATED aqueous extract
second precipitate

Fig. 1

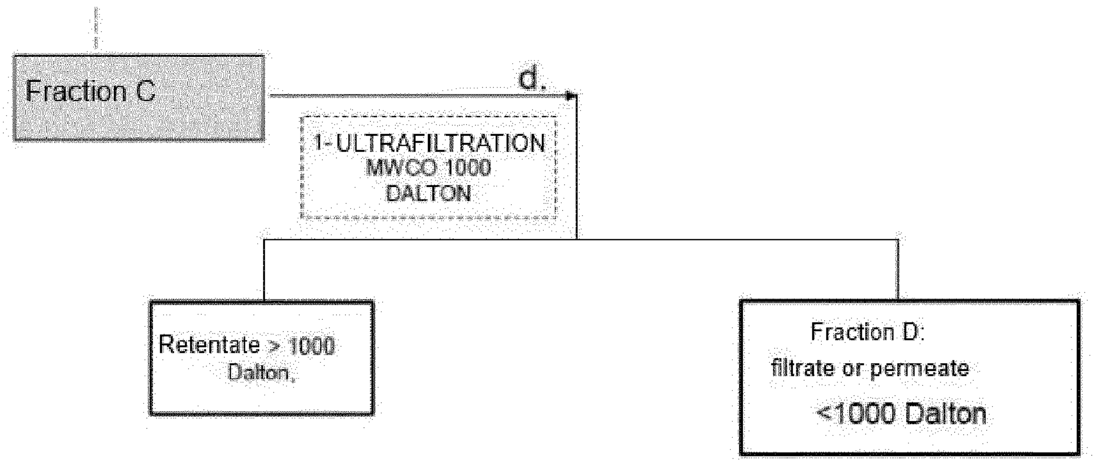
Fraction C
d.
1-ULTRAFILTRATION MWCO 1000 DALTON
Retentate > 1000 Dalton,
Fraction D: filtrate or permeate <1000 Dalton

Fig. 2

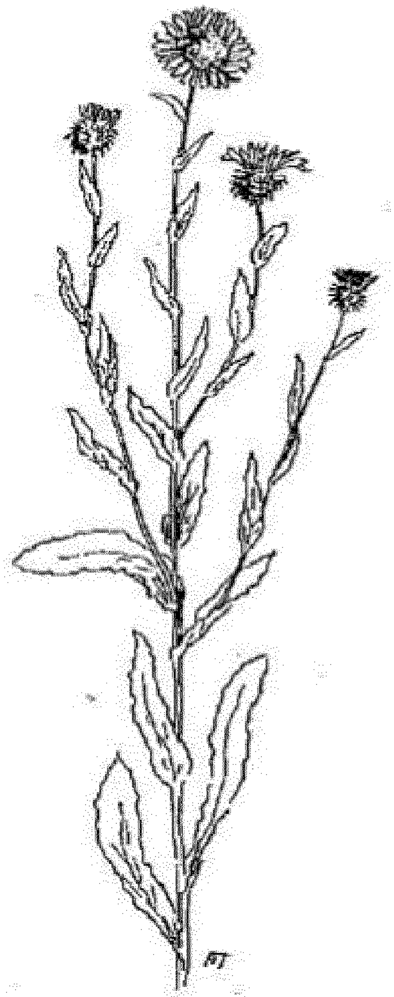

*Grindelia robusta* Nutt.,
Grindélia,

Fig. 3

Europäisches Patentamt
European Patent Office
Office européen des brevets

# EUROPEAN SEARCH REPORT

Application Number
EP 14 18 7604

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FR 2 953 140 A1 (ROCHER YVES BIOLOG VEGETALE [FR]) 3 June 2011 (2011-06-03)<br>* the whole document *<br>----- | 1-16 | INV.<br>A61P17/16<br>A61K36/28 |
| Y | US 8 491 941 B1 (LANGLEY WENDY S [US] ET AL) 23 July 2013 (2013-07-23)<br>* the whole document *<br>----- | 1-16 | |
| Y | TIMMERMANN B N ET AL: "Quantitative variation of grindelane diterpene acids in 20 species of North American Grindelia", BIOCHEMICAL SYSTEMATICS AND ECOLOGY, PERGAMON PRESS, GB,<br>vol. 15, no. 4, 15 July 1987 (1987-07-15), pages 401-410, XP023532286,<br>ISSN: 0305-1978, DOI: 10.1016/0305-1978(87)90053-6<br>[retrieved on 1987-07-15]<br>* the whole document *<br>----- | 1-16 | |
| Y | CANAVAN DON ET AL: "Successful treatment of poison oak dermatitis treated with Grindelia spp. (Gumweed)", JOURNAL OF ALTERNATIVE AND COMPLEMENTARY MEDICINE, MARY ANN LIEBERT, NEW YORK, NY, US,<br>vol. 11gr, no. 4,<br>1 August 2005 (2005-08-01), pages 709-710, XP002591706,<br>ISSN: 1075-5535<br>* the whole document *<br>-----<br>-/-- | 1-16 | TECHNICAL FIELDS SEARCHED (IPC)<br>A61K |

1 The present search report has been drawn up for all claims

| Place of search | Date of completion of the search | Examiner |
|---|---|---|
| Munich | 29 January 2015 | Greif, Gabriela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 18 7604

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KRESS H: "Grindelia (U.S.P.) - Grindelia", INTERNET CITATION, 12 March 2010 (2010-03-12), pages 1-5, XP002572927, Retrieved from the Internet: URL:http://www.henriettesherbal.com/eclectic/kings/grindelia.html [retrieved on 2010-03-12] * the whole document * ----- | 1-16 | |
| Y | Analise Ozols: "Grindelia squarrosa: An Ayurvedic View", , 22 March 2012 (2012-03-22), XP002725529, Retrieved from the Internet: URL:http://www.alandiashram.org/gurukula_blog/2012/03/grindelia-squarrosa-an-ayurvedic-view.html [retrieved on 2014-06-03] * the whole document * ----- | 1-16 | TECHNICAL FIELDS SEARCHED (IPC) |
| A | DIDRY N ET AL: "CHEMICAL COMPOSITION AND ANTI BACTERIAL ACTIVITY OF LEAVES OF VARIOUS SPECIES OF GRINDELIA", PLANTES MEDICINALES ET PHYTOTHERAPIE, ANGERS, FR, vol. 16, no. 1, 1 January 1982 (1982-01-01), pages 7-15, XP009178315, ISSN: 0032-0994 * the whole document * ----- | 1-16 | |

-/--

1

The present search report has been drawn up for all claims

| Place of search | Date of completion of the search | Examiner |
|---|---|---|
| Munich | 29 January 2015 | Greif, Gabriela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 18 7604

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GUILLERMO F. RETA ET AL: "Derivatives of grindelic acid: From a non-active natural diterpene to synthetic antitumor derivatives", EUROPEAN JOURNAL OF MEDICINAL CHEMISTRY, vol. 67, 1 September 2013 (2013-09-01), pages 28-38, XP055121330, ISSN: 0223-5234, DOI: 10.1016/j.ejmech.2013.06.013 * the whole document * ----- | 1-16 | |
| A | SMART ET AL: "The basics and underlying mechanisms of mucoadhesion", ADVANCED DRUG DELIVERY REVIEWS, ELSEVIER, AMSTERDAM, NL, vol. 57, no. 11, 3 November 2005 (2005-11-03), pages 1556-1568, XP027771403, ISSN: 0169-409X [retrieved on 2005-11-03] * the whole document * ----- | 1-16 | |
| | | | TECHNICAL FIELDS SEARCHED (IPC) |

1

The present search report has been drawn up for all claims

| Place of search | Date of completion of the search | Examiner |
|---|---|---|
| Munich | 29 January 2015 | Greif, Gabriela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 18 7604

This annex lists the patent family members relating to the patent documents cited in the above-mentioned European search report. The members are as contained in the European Patent Office EDP file on
The European Patent Office is in no way liable for these particulars which are merely given for the purpose of information.

29-01-2015

| Patent document cited in search report | | Publication date | Patent family member(s) | Publication date |
|---|---|---|---|---|
| FR 2953140 | A1 | 03-06-2011 | NONE | |
| US 8491941 | B1 | 23-07-2013 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **J.D. SMART.** *Advanced drug delivery reviews,* 2005, vol. 57, 1556-1568